# EUROPEAN PATENT APPLICATION

(11) **EP 1 498 145 A1**
(43) Date of publication of application: **19.01.2005**
(21) Application number: 03717715.1
(22) Date of filing: 24.04.2003
(51) Int. Cl.: A61K 48/00, A61K 38/00, A61K 45/00, A61P 11/06, A61P 37/02, A61P 43/00, C07K 14/47, C12N 1/15, C12N 1/19, C12N 1/21, C12N 5/10, C12N 15/09, G01N 33/15, G01N 33/50

(54) **DECOY FOR TREATING AND/OR PREVENTING Th2 CYTOKINE-ASSOCIATED ALLERGIC DISEASE, GATA3 MUTANT PROTEIN AND MEDICINAL COMPOSITIONS CONTAINING THE SAME**

(30) Priority: 24.04.2002 JP 2002123019; 19.12.2002 JP 2002368545
(71) Applicant: Ginkgo Biomedical Research Institute Co., Ltd., Tokyo 108-0071 (JP)
(72) Inventor: ARAI, Ken-ichi, Tokyo 153-0063 (JP); MIYATAKE, Shoichiro, Tokyo 112-0011 (JP); TAKEMOTO, Naofumi, Tokyo 142-0062 (JP)
(74) Representative: Bassil, Nicholas Charles
(86) International application number: PCT/JP2003/005276
(87) International publication number: WO 2003/090788

(57) **Abstract**

The present invention provides GATA3 decoys and GATA3 mutant proteins which can specifically suppress Th2 cytokine production and pharmaceutical compositions comprising such decoys and mutant proteins. This suppression is achieved by inhibiting binding of the GATA3 protein to the low affinity GATA3 binding sequence within the Th2 cytokine gene cluster, which causes suppression of the chromatin remodeling required for stable and sufficient expression of Th2 cytokines.

SEQUENCE LISTING

<110> Ginkgo Biomedical Research Institute Co., Ltd.

<120> Decoys for treating and/or preventing Th2 cytokine-related allergic
diseases, GATA3 mutant proteins, and pharmaceutical compositions comprising the
decoys and the proteins

<130> G2-A0204Y1P

<150> JP 2002-123019
<151> 2002-04-24

<150> JP 2002-368545
<151> 2002-12-19

<160> 14

<170> PatentIn Ver. 2.1

<210> 1
<211> 443
<212> PRT
<213> Mus musculus

<400> 1
Met Glu Val Thr Ala Asp Gln Pro Arg Trp Val Ser His His His Pro
  1               5                  10                  15

Ala Val Leu Asn Gly Gln His Pro Asp Thr His His Pro Gly Leu Gly
             20                  25                  30

His Ser Tyr Met Glu Ala Gln Tyr Pro Leu Thr Glu Glu Val Asp Val
         35                  40                  45

Leu Phe Asn Ile Asp Gly Gln Gly Asn His Val Pro Ser Tyr Tyr Gly
     50                  55                  60

Asn Ser Val Arg Ala Thr Val Gln Arg Tyr Pro Pro Thr His His Gly
 65                  70                  75                  80

Ser Gln Val Cys Arg Pro Pro Leu Leu His Gly Ser Leu Pro Trp Leu
                 85                  90                  95

Asp Gly Gly Lys Ala Leu Ser Ser His His Thr Ala Ser Pro Trp Asn
            100                 105                 110

Leu Ser Pro Phe Ser Lys Thr Ser Ile His His Gly Ser Pro Gly Pro
        115                 120                 125

Leu Ser Val Tyr Pro Pro Ala Ser Ser Ser Ser Leu Ala Ala Gly His
    130                 135                 140

Ser Ser Pro His Leu Phe Thr Phe Pro Pro Thr Pro Pro Lys Asp Val
145                 150                 155                 160

Ser Pro Asp Pro Ser Leu Ser Thr Pro Gly Ser Ala Gly Ser Ala Arg
                165                 170                 175

Gln Asp Glu Lys Glu Cys Leu Lys Tyr Gln Val Gln Leu Pro Asp Ser
            180                 185                 190

Met Lys Leu Glu Thr Ser His Ser Arg Gly Ser Met Thr Thr Leu Gly
        195                 200                 205

Gly Ala Ser Ser Ser Ala His His Pro Ile Thr Thr Tyr Pro Pro Tyr
    210                 215                 220

Val Pro Glu Tyr Ser Ser Gly Leu Phe Pro Pro Ser Ser Leu Leu Gly
225                 230                 235                 240

Gly Ser Pro Thr Gly Phe Gly Cys Lys Ser Arg Pro Lys Ala Arg Ser
                245                 250                 255

Ser Thr Glu Gly Arg Glu Cys Val Asn Cys Gly Ala Thr Ser Thr Pro
            260                 265                 270

Leu Trp Arg Arg Asp Gly Thr Gly His Tyr Leu Cys Asn Ala Cys Gly
        275                 280                 285

Leu Tyr His Lys Met Asn Gly Gln Asn Arg Pro Leu Ile Lys Pro Lys
    290                 295                 300

Arg Arg Leu Ser Ala Ala Arg Arg Ala Gly Thr Ser Cys Ala Asn Cys
305                 310                 315                 320

Gln Thr Thr Thr Thr Thr Leu Trp Arg Arg Asn Ala Asn Gly Asp Pro
                325                 330                 335

Val Cys Asn Ala Cys Gly Leu Tyr Tyr Lys Leu His Asn Ile Asn Arg
            340                 345                 350

Pro Leu Thr Met Lys Lys Glu Gly Ile Gln Thr Arg Asn Arg Lys Met
        355                 360                 365

Ser Ser Lys Ser Lys Lys Cys Lys Lys Val His Asp Ala Leu Glu Asp
    370                 375                 380

Phe Pro Lys Ser Ser Ser Phe Asn Pro Ala Ala Leu Ser Arg His Met
385                 390                 395                 400

Ser Ser Leu Ser His Ile Ser Pro Phe Ser His Ser Ser His Met Leu
                405                 410                 415

Thr Thr Pro Thr Pro Met His Pro Pro Ser Gly Leu Ser Phe Gly Pro
            420                 425                 430

His His Pro Ser Ser Met Val Thr Ala Met Gly
        435                 440


<210> 2
<211> 444
<212> PRT
<213> Homo sapiens

<400> 2

<210> 3
<211> 28

<212> DNA
<213> Artificial Sequence

<220>
<223> Description of Artificial Sequence:primer

<400> 3
gaggcctcat tatcttcatt catttctc                                          28


<210> 4
<211> 21
<212> DNA
<213> Artificial Sequence

<220>
<223> Description of Artificial Sequence:primer

<400> 4
gttagagata gcatcgcccc a                                                 21


<210> 5
<211> 28
<212> DNA
<213> Artificial Sequence

<220>
<223> Description of Artificial Sequence:primer

<400> 5
gaggtgtcct ctatctgatt gttagcaa                                          28


<210> 6
<211> 28
<212> DNA
<213> Artificial Sequence

<220>
<223> Description of Artificial Sequence:primer

<400> 6
aagccccatt atcttcattc atttctca                                          28


<210> 7
<211> 28
<212> DNA
<213> Artificial Sequence

<220>
<223> Description of Artificial Sequence:primer

<400> 7
ggaagtgtta cttctgctct aaaagctg                                          28


<210> 8
<211> 34
<212> DNA
<213> Artificial Sequence

<220>
<223> Description of Artificial Sequence:primer

<400> 8
acccggcgga tcccgggtgg tgcgtgtctg ggtg                                   34

<210> 9
<211> 37
<212> DNA
<213> Artificial Sequence

<220>
<223> Description of Artificial Sequence:primer

<400> 9
agacacgcac cacccgggat ccgccgggtc ggccagg                                37


<210> 10
<211> 28
<212> DNA
<213> Artificial Sequence

<220>
<223> Description of Artificial Sequence:primer

<400> 10
gtgggaggtt ttttctctag actagtct                                          28


<210> 11
<211> 29
<212> DNA
<213> Artificial Sequence

<220>
<223> Description of Artificial Sequence:primer

<400> 11
ggtagagtcc ctccctgcct tctgtgctg                                         29


<210> 12
<211> 35
<212> DNA
<213> Artificial Sequence

<220>
<223> Description of Artificial Sequence:primer

<400> 12
gcagggaggg actctaccat aaaatgaatg ggcag                                  35


<210> 13
<211> 35
<212> DNA
<213> Artificial Sequence

<220>
<223> Description of Artificial Sequence:primer

<400> 13
ttcatagtca ggggtcgaca gccttcgctt gggct                                  35


<210> 14
<211> 39
<212> DNA
<213> Artificial Sequence

<220>

<223> Description of Artificial Sequence:primer

<400> 14
aagcgaaggc tgtcgacccc tgactatgaa gaaagaagg 39

## Description

### Technical Field

The present invention relates to agents for treating and/or preventing Th2 cytokine-related allergic diseases, as well as methods for treating and/or preventing the same.

### Background Art

Th2 cytokine-related allergic disease accounts for a large proportion of healthcare expenditures in advanced countries. Globally, more than 150 million people suffer from asthma; accordingly, asthma therapeutic agents form a market of 230 billion yen and 760 billion yen per year in Japan and the United States, respectively. Allergic asthma is a chronic inflammatory pulmonary disease widely distributed among both children and adults, the major symptoms of which include airflow obstruction due to airway hyperresponsiveness and airway inflammation, and which may lead to death through status asthmaticus. In addition to environmental factors, genetic predisposition is considered to be involved in the development of the disease. However, detailed mechanisms of the development of the disease remain to be clarified.

Current therapeutic agents for asthma are roughly divided into two categories: 1) xanthine derivatives and β2 agonists (including steroids) for treating asthma attacks that target bronchodilation; and 2) anti-allergic agents with antiinflammatory effects as agents for long-term management, including chemical mediator release suppressants such as disodium cromoglycate, histamine antagonists, thromboxane inhibitors, and leukotriene antagonists. Therapeutic agents for asthma in the real meaning should be allergy-controlling agents that control the fundamental aspect of the disease, i.e., inflammatory reaction in the airway, and not those used for symptomatic treatment. Thus, development of therapeutic agents that belong to this new category, that accomplish prevention of the onset of allergies, is desired in the art. Steroids, for inhalation or internal use, are the most effective and powerful drugs used today. They promptly mitigate airway inflammation and exert a powerful anti-asthmatic effect through the suppression of immunocyte activity. However, steroids are not suitable for long-term use due to their strong side effects. Most of hitherto developed anti-allergic agents inhibit chemical mediators produced from inflammatory cells that trigger asthma attack at various levels. Recently, T helper type 2 (Th2) cells and cytokines produced therefrom were discovered to play a key role in the onset of allergies (see, *e.g.*, O'Garra, A. (1998) Immunity 8: 275; Glimcher, L.H. and Murphy, K.M. (2000) Genes Dev. 14: 1693; Murphy, K.M., et *al*. (2000) Annu. Rev. Immunol. 18: 451). Subsequently, antibodies and such that inhibit the function of Th2 cytokines, such as IL-4, IL-5, and IL-13, have been developed as therapeutic agents, clinical trial for which are currently in progress. However, many allergic diseases primarily result from repeated exposure to allergens. Therefore, suppression of allergic reaction targeting Th2 cytokines themselves may not be an effective therapeutic method.

Various groups of researchers have studied the differentiation mechanisms of Th2 cells and factors involved in such mechanism. As a result, the GATA3 protein was revealed not only to function as a transcription factor but also to play an important role in the differentiation and commitment of Th2 cells. Inhibitors of this process are considered to serve as a completely new type of therapeutic agent against all types of allergic diseases, including severe asthma for which withdrawal of steroid is difficult.

Helper T cells are divided at least into two subsets, Th1 and Th2, based on their pattern of cytokine production. This phenomenon was originally considered distinctive to *in vivo* cloned T cells. However, it was shown that the sensitivity to parasite in mice and the prognosis of actual disease such as leprosy in human are determined by which of the two subsets are activated. Thus, these subsets were recognized as important factors that determine the prognosis of infectious diseases. Th1 cells, which produce IFNγ and lymphotoxin, are said to be involved in cellular immunity, and are important for the elimination of intracellular pathogens such as bacteria and viruses. Inappropriate activation of Th1 cells induces autoimmune diseases. On the other hand, Th2 cells, which produce interleukin (IL)-4, 5, and 13, and so on, are involved in humoral immunity, and play roles in parasite disinfestation. However, overactivation of Th2 cells is known to cause allergic diseases such as asthma and atopic dermatitis.

An effective method for controlling Th2 cells based on this novel mechanism is essential for treating allergic diseases which have increased in recent years. Th2 cells differentiate from naive T cells due to antigen stimulation, and IL-4 is the major cytokine that induces the differentiation of Th2 cells. The binding of IL-4 to its receptor activates its downstream signal transduction pathway, JAK/STAT, and finally induces the expression of a Th2 cell specific transcription factor, the GATA3 protein. Th2 cytokine genes cluster on human chromosome 5 (mouse chromosome 11), and genes encoding IL-4, IL-13, and IL-5 exist within the 160 Kb of region q23-31. For a long time, it has been observed that the transcription of the Th2 cytokine gene group is simultaneously turned "on" upon the differentiation of Th2 cells. However, until recently, the underlying mechanism had not been revealed. In that vein, the present inventors recently discovered a region that controls the expression of the Th2 cytokine genes within this gene cluster (Takemoto, N., *et al.* (1998) Int. Immunol. 10: 1981-1985; Takemoto, N., *et al.* (2000) J. Immunol. 165: 6687-6691).

This region (HSS1, 2, and 3) shows good correspondence with CNS-1, one of the 15 regions (CN-1 to CN-15) whose nucleotide sequences are well-preserved among various mammals. Deletion of this site impaired expression of IL-4, IL-5, and IL-13 in animal experiments. Therefore, it was demonstrated that this region has a Locus Control Region (LCR)-like function, which is well examined in the globin gene. The present inventors also demonstrated that this region (CNS-1/HSS1, 2) has a recognition sequence for GATA3 protein and that a GATA3 protein actually binds to this sequence (Takemoto, N., *et al*. (2000) supra).

### Disclosure of the Invention

The present invention relates to pharmaceutical compositions for treating and/or preventing Th2 cytokine-related allergic diseases. In particular, it relates to GATA3 decoys and GATA3 mutant proteins. Furthermore, the present invention provides GATA3 decoys and GATA3 mutant proteins that specifically suppress chromatin remodeling at the site on the chromosome where the Th2 cytokine gene cluster required for the production of Th2 cytokines exists. Moreover, it provides a method for screening agents useful for the treatment and/or prevention of Th2 cytokine-related allergic diseases that target suppression of chromatin remodeling as described above.

*In vitro* induction of Th2 cells requires multiple antigen stimulations of naive T cells in the presence of IL-4. Through these repeated stimulations, the differentiation of Th2 cells reaches the final stage and thereafter, does not change its phenotype. This phenomenon is called the "commitment of cells" and is analogous to the process whereby a hematopoietic stem cell commits to become a differentiated leukocyte, which, in turn, never changes to an erythrocyte. Th2 cells activated in allergic diseases such as asthma were previously believed to be at this final stage; thus, it was presumed to be impossible to change its phenotype and its cytokine production activity. However, the present inventors have discovered and reported that the above-mentioned transcription factor GATA3 protein changes the character of a cell and forces the cell to produce other cytokines by changing the chromatin structure to accomplish induction of redifferentiation into Th1 cells, a act which had previously been considered impossible (Lee, *et al*. (2000) J. Exp. Med. 192: 105; Takemoto, N., *et al.* (2000) *supra*). Hence, low molecular compounds and decoy nucleotides that specifically inhibit this function of the GATA3 transcription factor protein enable the transformation of Th2 cells that are the basis of allergies. Accordingly, they represent potential candidates for novel therapeutic agents that differ from existing drugs that either involve symptomatic treatment against inflammation or suppress immune cells as a whole (e.g., steroids).

The present inventors have found that the master regulator function of the GATA3 protein is involved in the change of chromatin structure that accompanies the commitment of naive T cells to Th2 cells. Furthermore, they have revealed that this function is exhibited through the recognition of a nucleotide sequence by a specific domain of the GATA3 protein, which nucleotide sequence differs from the DNA sequence required for the general function of the GATA3 protein. Finally, they have discovered that the GATA3 protein serves as a target for a novel therapeutic agent for asthma.

Based on these findings, the DNA sequence to which the GATA3 protein binds to exert its function as the master regulator has been specified in the present invention (SEQ ID NO: 3 shows the sense chain sequence of mouse, and SEQ ID NO: 6 that of human; see also the HSS2 sequence depicted on the top of Fig. 1 for the mouse sequence). It was revealed that the binding of GATA3 protein to this sequence has a different binding mode compared to that to other GATA3 binding sequences within the transcription regulatory regions of the IL-5 gene and TCRα gene. Namely, it was found that the region within the GATA3 protein required for the binding to this sequence differs from the one required for the binding to other GATA3 binding sequences within the transcription regulatory regions of genes, such as the IL-5 and TCRα genes, that undergo transcription activation by GATA3 proteins. Furthermore, differences in binding affinity were also discovered.

Thus, the present invention encompasses the following embodiments, numbered 1 to 30, useful for treating and/or preventing asthma:
[1] a GATA3 decoy for treating and/or preventing Th2 cytokine-related allergy diseases;
[2] the GATA3 decoy according to [1], wherein the decoy suppresses chromatin remodeling within a Th2 cytokine gene cluster region by inhibiting the binding of a GATA3 protein to an HSS2 sequence in the genome and thereby suppresses the production of one or more Th2 cytokines in T cells;
[3] the GATA3 decoy according to [2], wherein the decoy does not inhibit the binding of a GATA3 protein to a GATA3 binding sequence in a transcription regulatory region of a gene which is transcriptionally activated by the GATA3 protein;
[4] the GATA3 decoy according to [2], wherein the decoy inhibits at a lower level the binding of a GATA3 protein to a GATA3 binding sequence in a transcription regulatory region of a gene which is transcriptionally activated by the GATA3 protein as compared to the binding of the GATA3 protein to an HSS2 sequence in the genome;
[5] the GATA3 decoy according to [1], wherein the decoy specifically inhibits the binding of a GATA3 protein to an HSS2 sequence in the genome;
[6] the GATA3 decoy according to any one of [1] to [5], wherein the decoy comprises a double stranded oligonucleotide, or derivative thereof, comprising a sequence set forth in SEQ ID NO: 3, 4, 5, or 6;
[7] the GATA3 decoy according to any one of [1] to [6], wherein the decoy effectively inhibits the binding of a GATA3 protein to an HSS2 sequence in a T cell genome, further wherein the decoy can be administered at a concentration such that it inhibits at a lower level the binding of the GATA3 protein to a GATA3 binding sequence in a transcription regulatory region of a gene which is transcriptionally activated by the GATA3 protein as compared to the binding of the GATA3 protein to the HSS2 sequence in the genome;
[8] a GATA3 mutant protein or derivative thereof, wherein:
   a) the amino acid sequence of the C finger region within a wild type GATA3 protein is conserved; or
   b) one or more amino acids are deleted, substituted, or inserted in the amino acid sequence of the C finger region within the wild type GATA3 protein yet the binding ability to HSS2 sequence is maintained;
[9] the GATA3 mutant protein or derivative thereof according to [8], wherein one or more amino acids are deleted, substituted, or inserted in the amino acid sequence of the transactivation region within the wild type GATA3 protein such that the ability of the GATA3 protein to induce chromatin remodeling is lost;
[10] the GATA3 mutant protein or derivative thereof according to [9], wherein at least ten amino acid residues are deleted in the amino acid sequence of the transactivation region within the wild type GATA3 protein;
[11] the GATA3 mutant protein or derivative thereof according to any one of [8] to [10], wherein one or more amino acids are further deleted, substituted, or inserted in the amino acid sequence of the N finger region within the wild type GATA3 protein such that the ability to bind to a DNA comprising a GATA3 binding sequence within a transcription regulatory region of a gene which is transcriptionally activated by the GATA3 protein is lost;
[12] the GATA3 mutant protein or derivative thereof according to any one of [8] to [10], wherein at least one amino acid residue is deleted in the amino acid sequence of the N finger region within the wild type GATA3 protein;
[13] a GATA3 mutant protein or derivative thereof, wherein at least ten amino acid residues are deleted in the amino acid sequence of the transactivation region and at least one amino acid residue is deleted in the amino acid sequence of the N finger region within the wild type GATA3 protein;
[14] a mouse GATA3 mutant protein or derivative thereof, wherein amino acids 29 to 168 in the amino acid sequence of the mouse wild type GATA3 protein set forth in SEQ ID NO: 1 are deleted;
[15] a human GATA3 mutant protein or derivative thereof, wherein amino acids 29 to 169 in the amino acid sequence of the human wild type GATA3 protein set forth in SEQ ID NO: 2 are deleted;
[16] the GATA3 mutant protein or derivative thereof according to [14], wherein at least amino acids 280 to 287 in the amino acid sequence of the N finger region within the mouse wild type GATA3 protein set forth in SEQ ID NO: 1 are further deleted;
[17] the human GATA3 mutant protein or derivative thereof according to [15], wherein at least amino acids 281 to 288 in the amino acid sequence of the N finger region within the human wild type GATA3 protein set forth in SEQ ID NO: 2 are further deleted;
[18] the GATA3 mutant protein or derivative thereof according to any one of [8] to [17], wherein the protein or derivative has the ability to bind to the HSS2 sequence but suppress Th2 cytokine production in T cells;
[19] the GATA3 mutant protein or derivative thereof according to [18], wherein the ability to bind to a DNA comprising a GATA3 binding sequence within a transcription regulatory region of a gene which is transcriptionally activated by a GATA3 protein is lost;
[20] a nucleic acid encoding the GATA3 mutant protein according to any one of [8] to [19] ;
[21] a vector comprising the nucleic acid according to [20] ;
[22] the vector according to [21], wherein the vector is an expression vector;
[23] a host cell comprising the vector according to [21] or [22] ;
[24] a pharmaceutical composition for treating and/or preventing Th2 cytokine-related allergic diseases, wherein the composition suppresses the production of one or more Th2 cytokines upon administered to a mammal by suppressing chromatin remodeling within a Th2 cytokine gene cluster region due to the inhibition of binding of a GATA3 protein to the HSS2 sequence in the genome;
[25] the pharmaceutical composition according to [24], wherein the pharmaceutical composition comprises one or more of the GATA3 decoys according to any one of [1] to [7] ;
[26] the pharmaceutical composition according to [24], wherein the pharmaceutical composition comprises one or more of the GATA3 mutant proteins according to any one of [8] to [19] ;
[27] the pharmaceutical composition according to [24], wherein the pharmaceutical composition comprises one or more of the nucleic acids encoding a GATA3 mutant protein according to [20];
[28] the pharmaceutical composition according to [24], wherein the pharmaceutical composition comprises one or more of the vectors according to [21] or [22];
[29] a method of screening for a drug for treating and/or preventing Th2 cytokine-related allergic diseases, wherein the method comprises the steps of:
   a) incubating a wild type GATA3 protein or a GATA3 mutant protein which possesses the ability to bind to an HSS2 sequence with a dsDNA or derivative thereof which comprises the HSS2 sequence, with or without a test compound, under conditions that allow for the binding of the wild type GATA3 protein or the GATA3 mutant protein and the dsDNA or derivative thereof;
   b) detecting the binding of the wild type GATA3 protein or the GATA3 mutant protein to the dsDNA or derivative thereof; and
   c) identifying a test compound which is able to inhibit said binding; and
[30] the method according to [29], wherein the method further comprises the steps of:
   d) incubating the wild type GATA3 protein or a GATA3 mutant protein which possesses the ability to bind to a GATA3 binding sequence within a transcription regulatory region of a gene with a dsDNA or derivative thereof which comprises the GATA3 binding sequence within the transcription regulatory region of the gene which is transcriptionally activated by a GATA3 protein, with or without the test compound, under conditions that allow for the binding of the wild type GATA3 protein or GATA3 mutant protein and the dsDNA or derivative thereof;
   e) detecting the binding of the wild type GATA3 protein or GATA3 mutant protein to the dsDNA or derivative; and
   f) identifying the test compound which inhibits the binding detected in step e) at a lower level as compared to the binding detected in step b).

As used herein, the term "Th2 cytokine" encompasses interleukin (IL)-4, IL-13, and IL-5 produced by Th2 cells. The production of these cytokines is characteristic of Th2 cells and is not observed in Th1 cells.

As used herein, the term "Th2 cytokine-related allergic disease" encompasses allergic diseases in which the above-mentioned Th2 cytokine production is involved in the mechanism of onset, such as asthma, atopic dermatitis, atopic asthma, and pollinosis.

As used herein, the term "GATA3 decoy" encompasses all compounds which are able to specifically antagonize the DNA binding site of a GATA3 protein. These compounds include, but are not limited to, nucleic acids and nucleic acid derivatives. Preferred examples of GATA3 decoys include oligonucleotides comprising the GATA3 binding sequence of the transcription regulatory region of the IL-5 gene (*e.g.*, the mouse-derived oligonucleotide shown in SEQ ID NO: 4 and corresponding human oligonucleotide); oligonucleotides comprising the GATA3 binding sequence of the transcription regulatory region of the TCRα gene (*e.g.*, the mouse-derived oligonucleotide shown in SEQ ID NO: 5 and corresponding human oligonucleotide); and oligonucleotides comprising the GATA3 binding sequence of the HSS2 sequence (*e.g.*, the mouse-derived oligonucleotide shown in SEQ ID NO: 3 and corresponding human oligonucleotide shown in SEQ ID NO: 6). As shown in the Examples below, the binding specificity of an oligonucleotide which comprises the GATA3 binding sequence of the transcription regulatory region of the IL-5 gene (*e.g.*, the oligonucleotide shown in SEQ ID NO: 4) or an oligonucleotide which comprises the GATA3 binding sequence of the transcription regulatory region of the TCRα gene (*e.g.*, the oligonucleotide shown in SEQ ID NO: 5) to the GATA3 protein is higher than the binding specificity of the GATA3 protein to the GATA3 protein sequence within the HSS2 sequence. Therefore, the inhibition of GATA3 protein binding to the HSS2 sequence can be accomplished at lower concentrations. That means, when an oligonucleotide comprising a GATA3 binding sequence with high binding affinity, or a derivative or modification thereof, is administered to patients as a decoy, the binding of the GATA3 protein to the HSS2 sequence, which has low binding affinity, is inhibited. However, as compared to the inhibition of binding between the HSS2 sequence and the GATA3 protein which has low affinity, the high affinity binding of the GATA3 protein with other GATA3 binding sequences is difficult to inhibit due to their high affinity. Since binding between the HSS2 sequence and the GATA3 protein which is necessary for chromatin remodeling has a lower affinity as compared to binding between other GATA3 binding sequences and the GATA3 protein, there should be a certain concentration of decoy that enables selective inhibition of binding between the HSS2 sequence and the GATA3 protein. Those skilled in the art can easily estimate such a concentration by general in vitro experimental procedures using techniques such as gel shift assay and cell culture.

Oligonucleotides used as decoys can be DNA or RNA, and they can comprise modified nucleotides and other compounds. Furthermore, the oligonucleotide can comprise mutations, such as sequence deletions, substitutions, and/or additions at one or more sites so long as the ability of the decoy oligonucleotide to bind to the GATA3 protein is not lost. Moreover, the oligonucleotide can be single- or double-stranded, linear or circular. More preferably, the decoy oligonucleotide is a double-stranded oligonucleotide and comprises one or more GATA3 binding sequences. SEQ ID NOS: 3, 4, 5, and 6 indicate sense-strands of respective sequences, and when used as a double-strand, each oligonucleotide annealed with its complementary strand is used. In order to suppress degradation before and after administration, the oligonucleotide can be modified to contain a thiophosphodiester bond by substituting the oxygen atom of a phosphodiester site with a sulfur atom. Moreover, a phosphodiester site can be substituted with a methylphosphate which does not possess a charge. Alkylation, acylation, or other chemical modifications can also be performed. GATA3 decoys of the present invention can be synthesized by conventional chemical or biochemical methods. When the decoy is a nucleic acid, it can be synthesized using any gene manipulation technique (including restriction enzyme digestion and gene recombination methods), PCR methods, or nucleic acid synthesizers. These methods can easily be performed by those skilled in the art.

As used herein, the HSS2 sequence is a sequence which comprises a GATA3 binding sequence that is located near a DNase I hypersensitive site (HSS2 site) (this site is easily cut by DNase I) within the region called CNS-1, located between the IL-4 and IL-13 genes in the genome. For example, the sequence (the sense-strand is shown in SEQ ID NO: 3) indicated in the HSS2 column on the top of Fig. 1 is the mouse HSS2 sequence. The human HSS2 sequence is indicated in SEQ ID NO: 6 (only sense-strand is shown). As depicted, the HSS2 sequence is able to bind to the GATA3 protein, such binding being necessary for chromatin remodeling which is, in turn, required for Th2 cytokine production in T cells.

As used herein, the term "GATA3 binding sequence" refers to a DNA sequence to which the GATA3 protein specifically binds and comprising a GATA3 protein binding consensus sequence (A/T)GATA(A/G). The GATA3 protein is a transcription factor that affects many genes. It is known that transcription regulatory regions of genes which are transcriptionally activated by the GATA3 protein contain a GATA3 binding sequence and the binding of the GATA3 protein to the sequence is necessary for transcriptional activation of the genes. Examples of such genes include, but are not limited to, the IL-5 gene, the TCRα gene, and CD8. Binding of the GATA3 protein to the HSS2 sequence required for the chromatin remodeling described above is also contributed by binding of the GATA3 protein to the GATA3 binding sequence within the HSS2 sequence. Binding of the GATA3 protein to the HSS2 sequence has a lower binding affinity for the GATA3 protein as compared to the binding of the GATA3 protein to the GATA3 binding sequences within the above-mentioned transcription regulatory regions of genes. Moreover, it is believed to carry out only modification of chromatin structure by chromatin remodeling, but not the transcriptional activation of specific genes. Therefore, the GATA3 binding sequence within the HSS2 sequence is not included in the term "GATA3 binding sequences within the transcription regulatory regions of genes" in the context of the present description.

As used herein, the term "chromatin remodeling" refers to the modification of the chromatin structure in T cells resulting from the binding of the GATA3 protein to the region where the Th2 cytokine gene cluster is located in the chromosome which causes the chromatin structure to be modified such that Th2 cytokine genes are transcribed by a gene specific transcription regulatory mechanism. That is, chromatin remodeling does not occur when binding between the GATA3 protein and the HSS2 sequence in the genome is inhibited or suppressed, and Th2 cytokines can not be produced stably or sufficiently.

The amino acid sequences of GATA3 proteins from various animal species are known. For example, the amino acid sequences of mouse and human wild type GATA3 proteins are indicated in SEQ ID NO: 1 and 2, respectively. These sequences are highly conserved; only Ala at amino acid number 38 in human is missing in mouse, which causes a single amino acid shift in the C-terminal side of the amino acid number 38. The basic structure of the GATA3 protein is conserved in all species and comprises a transactivation region containing two transactivation domains, transactivation domain I (amino acids 30 to 74 in human and 30 to 73 in mouse) and transactivation domain II (amino acids 131 to 214 in human and 130 to 213 in mouse), at the N-terminus. The C-terminal side of the sequence contains two zinc finger regions. The zinc finger region which is closer to N-terminus is called the N finger (amino acids 264 to 288 in human and 263 to 287 in mouse), and the other, which is closer to C-terminus, is called the C finger (C finger: amino acids 305 to 326 in human and 304 to 325 in mouse). Each of these zinc finger regions contains four cysteine residues and the deletion or substitution of any one of the cysteine residues can cause a loss of function. Binding to the GATA3 binding sequences within the transcription regulatory regions of genes requires both N finger and C finger regions and, furthermore, the transactivation region is required for the induction of transcription activation after binding. On the other hand, binding of the GATA3 protein to the HSS2 sequence, which is required for chromatin remodeling, requires only the C finger region; the N finger region is not necessary. Accordingly, the present invention encompasses GATA3 mutant proteins in which the amino acid sequence of the C finger region is preserved or one or more amino acids are deleted, substituted, or added so long as the ability of the protein to bind to HSS2 necessary for the induction of chromatin remodeling is maintained after the deletion, substitution, or addition. Therefore, while deletions substitutions, or insertions of one or more amino acid residues in the N finger region may result in loss of the ability of the mutant protein to bind to the GATA3 binding sequences within the transcription regulatory regions of genes, the ability of the mutant protein to bind to the HSS2 sequence is not affected by such deletions, substitutions, or insertions. Preferred GATA3 mutant proteins which are able to bind to the HSS2 sequence but do not have ability to bind to the GATA3 binding sequences within the transcription regulatory regions of genes comprise a deletion, substitution, or insertion of at least one residue, preferably 5 residues or more, more preferably 8 residues or more, even more preferably 10 residues or more in the amino acid sequence of N finger region. For example, the mouse delN GATA3 mutant protein used in the Examples has lost binding ability to the GATA3 binding sequences within the transcription regulatory regions of the IL-5 and TCRα genes but can induce chromatin remodeling because it maintains the ability to bind to the HSS2 sequence.

Moreover, the transactivation region is required for the induction of chromatin remodeling after binding, and therefore, GATA3 mutant proteins having a deletion, substitution, or addition of one or more amino acids (preferably 10 residues or more) in this region may lose the ability to induce chromatin remodeling yet retain the ability to bind to the GATA3 binding sequence. Preferred GATA3 mutant proteins which can bind to the GATA3 binding sequence but can not induce chromatin remodeling comprise a deletion, substitution, or insertion of at least one residue, preferably 10 residues or more, more preferably 50 residues or more, even more preferably 100 residues or more in the amino acid sequence within the transactivation region. For example, the mouse delTA GATA3 mutant protein used in the Examples possesses the ability to bind to the GATA3 binding sequence but has lost the ability to induce chromatin remodeling.

Therefore, GATA3 mutant proteins which comprise a deletion, substitution, or insertion of one residue or more, preferably 5 residues or more, more preferably 8 residues or more, and even more preferably 10 residues or more in the amino acid sequence within the N finger, and moreover, comprise a deletion, substitution, or insertion of one residue or more, preferably 10 residues or more, more preferably 50 residues or more, and most preferably 100 residues or more in the amino acid sequence within the transactivation region, may lose binding ability to the GATA3 binding sequences within the transcription regulatory regions of genes, and moreover, may maintain the ability to bind to the HSS2 sequence but lose the ability to induce chromatin remodeling. When such GATA3 mutant proteins are exogenously introduced into the nucleus (by either the gene manipulation method or the protein injection method), they can inhibit binding of endogenous GATA3 protein to the HSS2 sequence by binding to the HSS2 sequence without inducing chromatin remodeling. Moreover, these GATA3 mutant proteins do not bind to the GATA3 binding sequences within the transcription regulatory regions of genes including IL-5 and TCRα. Therefore, these GATA3 mutant proteins do not inhibit binding of endogenous GATA3 protein to them. That is, it is possible to selectively inhibit the induction of chromatin remodeling among the functions of the GATA3 protein, thereby suppressing Th2 cytokine production. An example of such a GATA3 mutant protein is a mouse GATA3 mutant protein in which amino acid residues 29 to 168 within the amino acid sequence of mouse wild type GATA3 protein of SEQ ID NO: 1 and at least amino acid residues 280 to 287 (*e.g.*, 263 to 287) in the N finger region are deleted. Another example includes a mouse GATA3 mutant protein in which the amino acid residues in human corresponding to the mentioned deleted amino acid residues, i.e., amino acid residues 29 to 169 within the amino acid sequence of human GATA3 protein of SEQ ID NO: 2 and at least amino acid residues 281 to 288 (*e.g.*, 264 to 288) in N finger region, are deleted.

The above-mentioned GATA3 mutant proteins and their derivatives which can suppress the production of Th2 cytokines can be used as pharmaceutical compositions to treat and/or prevent Th2 cytokine-related allergic diseases. Therefore, pharmaceutical compositions comprising the mentioned GATA3 mutant proteins are also within the scope of the present invention. In this case, the term "GATA3 mutant protein derivatives" encompasses modified or altered GATA3 mutant proteins that retain the activities of the GATA mutant protein such as the ability to bind to the HSS2 sequence, gene transcription activity, and the ability to induce chromatin remodeling.

A method to produce GATA3 mutant proteins of the present invention can easily be realized by referencing previously reported amino acid sequences of GATA3 proteins (see SEQ ID NO: 1 and SEQ ID NO: 2 for mouse and human GATA3 proteins, respectively) by those skilled in the art. Such proteins can be produced according to conventional techniques for producing nucleic acids encoding certain amino acid sequences. Various genetic manipulation methods, such as the PCR method, various mutagenesis methods, restriction enzyme digestion, and ligation using DNA ligase, may be appropriately used. See, *e.g.*, Sambrook, *et al.* (Molecular Cloning: A Laboratory Manual. 2nd ed., Cold Spring Harbor Laboratory, Cold Spring Harbor Laboratory Press, Cold Spring Harbor, NY, 1989). For example, a nucleic acid encoding an amino acid sequence of a wild type GATA3 protein can be cloned from a cDNA library derived from a corresponding animal species by the PCR or hybridization methods. The nucleic acid may be altered by the above-mentioned techniques so that it codes for a desired mutant. The mutant can be expressed by cloning the nucleic acid encoding the desired mutant into an appropriate expression vector and transforming an appropriate host (*e.g.*, *E. coli* and mammalian cells). The mutant protein can easily be obtained from the transformed cells by those skilled in the art. Therefore, vectors (*e.g.*, retroviral vectors) comprising the above-mentioned nucleic acid and host cells comprising the vectors are also within the scope of the present invention. Such a mutant can be expressed in T cells by introducing, in the form that can be delivered to T cells, the expression vector to which a nucleic acid encoding the mutant is functionally ligated.

The "pharmaceutical compositions" of the present invention may comprise pharmaceutically acceptable carriers, and additives such as thickeners, stabilizers, and/or solubilizers can be added as necessary. Examples of such carriers and additives include, but are not limited to, lactose, citric acid, stearic acid, magnesium stearate, sucrose, starch, talc, gelatin, agar, vegetable oil, and ethylene glycol. Moreover, flavoring agents, preservatives, coloring agents, sweeteners, and such can also be added as necessary depending on the administration method. The term "pharmaceutically acceptable" is readily understood by those skilled in the art, and indicates being approved by the authority of each country, or listed in the pharmacopoeia of each country or generally accepted pharmacopoeias concerning the animal, mammal, and especially human use. An amount effective for treating and/or preventing Th2 cytokine-related diseases can be administered through many suitable routes including, but not limited to, oral inhalation or aspiration, nasal inhalation or aspiration, oral administration, non-oral administration, such as intravenous injection, intraperitoneal injection, intramuscular injection, subcutaneous injection, percutaneous injection, and continuous infusion, and local administration, including topical application. Moreover, it can be administered with delivery vehicles to facilitate delivery to specific tissues or cell types (preferably thymus or T cells in the present invention). The pharmaceutical compositions of the present invention can be formulated into suitable forms for various applications, such as solution, suspension, emulsion, syrup, liposome, tablet, pill, powder, granule, capsule, aerosol, spray, ointment, and cream. Furthermore, the compositions may be provided together with ampules which contain sterile water, physiological saline, or buffer for injection. These solutions are mixed with the compositions prepared as freeze-dried powders or pellets in a single or multiple doses to prepare a desired concentration of the composition before the administration. The effective dose of the pharmaceutical compositions of the present invention for treatment and/or prevention of Th2 cytokine-related diseases can be determined by medical personnel using standard clinical techniques. Furthermore, in vivo assays, using disease model animals or the like, or in vitro assays can be freely used to determine the range of the optimal dose. The exact dose to be used is also dependent on the administration route and severity of the disease, and should be determined by the executor according to each patient's symptoms. As used herein, the term "patient" indicates any mammal, preferably human.

The preferred formulation for a pharmaceutical composition comprising as the active ingredient a GATA3 decoy which is a nucleic acid and/or its derivative or modification, and/or a GATA3 mutant protein includes liposomes which are generally used for gene therapy. Liposomes can be produced by methods described in numerous literature such as Szoka, F. *et al.* (Biochim. Biophys. Acta, Vol. 601 559 (1980)) and Brunner, J. *et al.* (Biochim. Biophys. Acta, Vol. 455 322 (1976)).

Moreover, the present invention also includes a method of screening for drugs to treat and/or prevent Th2 cytokine-related allergic diseases using as a target a compound which can inhibit the binding of the GATA3 protein to the HSS2 sequence. The compounds to be screened include any substances, including substances existing in living bodies, such as nucleic acids and proteins, without regard to molecular weight. That is, the decoys and GATA3 mutant proteins and their derivatives of the present invention can also be screened. In this method, a test compound which can inhibit the binding of wild type GATA3 protein to the HSS2 sequence can be identified by incubating wild type GATA3 protein and dsDNA or its derivative which comprises the HSS2 sequence with or without the test compound and detecting the binding between the wild type GATA3 protein and the dsDNA or its derivative under conditions that allow the wild type GATA3 protein to bind to the dsDNA or its derivative (the conditions can be those used for general gel shift assay and such. See, *e.g.*, Lee *et al.* (1998) J. Immunol. 160: 2343). In this method, GATA3 mutant proteins which possess the ability to bind to the HSS2 sequence (*e.g.*, delTA and delN GATA3 mutant proteins in the present description, though the present invention is not limited thereto) can be used instead of wild type GATA3 protein.

According to the above method, test compounds which do not inhibit binding of wild type GATA3 protein to the GATA3 binding sequence within the transcription regulatory region of a gene which is transcriptionally activated by GATA3 protein, such as IL-5 and/or TCRα (though the present invention is not limited thereto), or which inhibit it at a lower level as compared to the inhibition for the binding of wild type GATA3 protein to the HSS2 sequence, can be further identified by incubating dsDNA or its derivative which comprises a GATA3 binding sequence within the transcription regulatory region of the gene and wild type GATA3 protein, with or without the test compound, under the conditions that allow wild type GATA3 protein to bind to the dsDNA or its derivative. Thus, a compound which can specifically inhibit only the binding of the GATA3 protein to the HSS2 sequence, among GATA3 binding sequences, can be identified. In this process, GATA3 mutant proteins (*e.g.*, delTA GATA3 mutant protein in the present description, though the present invention is not limited thereto) having the ability to bind to the GATA3 binding sequences within the transcription regulatory regions of genes can be used instead of wild type GATA3 protein.

The detection and comparison of the degree of binding inhibition in the above-mentioned methods can easily be performed by labeling dsDNA or its derivative used for the above-mentioned method with various labeling compounds, such as radioactive, fluorescent, and chemiluminescent compounds. The selection of an appropriate labeling compound and method to label and compare the level of binding inhibition can easily be performed by those skilled in the art using various methods, such as conventional gel shift assay or a method wherein the GATA3 protein is bound to a solid support. Moreover, dsDNA derivatives which comprise the HSS2 sequence include derivatives and modifications as described in the above-mentioned decoys.

### Brief Description of the Drawings

Fig. 1 is a series of photographs and diagrams depicting the results of gel shift assays. DNA used in each assay is indicated on the top. The delN mutant protein can bind to the GATA3 binding sequences within the IL-5 promoter and TCRα enhancer; however, it cannot bind to the GATA3 binding sequence within HSS2. The delC mutant protein does not bind to any GATA3 binding sequence and the delTA mutant protein binds to all GATA3 binding sequences.
Fig. 2 is a series of photographs and diagrams depicting the results of DNase I hypersensitivity assays of the region between the IL-13 gene and IL-4 gene in transformed T cells expressing each GATA3 mutant protein.
Fig. 3 is a series of photographs and diagrams depicting the results of DNase I hypersensitivity assays of IL-4 gene coding region in transformed T cells expressing each GATA3 mutant protein.
Fig. 4 depicts IFNγ, IL-4, and IL-5 expression measured by the immunochemical flow cytometric method in transformed Th1 cells expressing each GATA3 mutant protein. The delTA and delC mutants showed expression patterns similar to that of Th1 cells for all the cytokines tested. However, in transformed delN expressing cells, IFNγ expression was suppressed and IL-4 expression was increased as compared to IFNγ and IL-4 expression in uninfected cells. This was similar to wild type GATA3 protein expressing cells, and this tendency was the same as that observed in Th2.
Fig. 5 depicts the results of competitive assays in the binding of the HSS2 sequence and the GATA3 binding sequences within the gene transcription regulatory region to GATA3 protein. The left panel depicts the results of an electrophoretic mobility shift assay (EMSA) using cell extracts collected from COS cells expressing GATA3 and a probe comprising the HSS2 sequence (HSSGATA sequence). The right panel depicts the results of an electrophoretic mobility shift assay (EMSA) using cell extracts collected from COS cells expressing GATA3 and a probe comprising the GATA3 binding sequence within the gene transcription regulatory region (TCRαGATA sequence).
Fig. 6 is a series of photographs depicting the results of gel shift assays comparing the binding affinity of GATA3 protein to the HSS2 sequence (CNS-1) and the GATA3 binding sequence within the gene transcription regulatory region (TCRα enhancer sequence).

### Best Mode for Carrying out the Invention

The present invention is illustrated in detail below with reference to Examples, but it is not to be construed as being limited thereto.

### [Example 1] Construction of genes encoding the wild type GATA3 protein and GATA3 mutant proteins

Wild type GATA3 protein and various GATA3 mutant proteins were prepared. Mouse GATA3 protein cDNA, retrovirus vector pMXI, and mammalian cell expression vector pME18S were provided by Professor Yamamoto (University of Tsukuba), Professor Kitamura (The Institute of Medical Science, The University of Tokyo), and Associate Professor Maruyama (Tokyo Medical and Dental University), respectively. In order to introduce the genes into T cells, wild type and mutant GATA3 cDNAs were inserted between EcoRI and NotI sites of pMXI which expresses green fluorescent protein (GFP) bicistronically. The cDNAs were also inserted into pME18S at EcoRI and NotI sites in order to express them in COS cells.

The delTA mutant has a deletion in the transcription activation region (lacking amino acids 29 to 168). Two PCR fragments comprising this deletion were generated using GATA3 protein-encoding cDNA as a template. The primer combinations of primer 1: 5'-GGAAGTGTTACTTCTGCTCTAAAAGCTG-3' (SEQ ID NO: 7)/primer 2: 5'-ACCCGGCGGATCCCGGGTGGTGCGTGTCTGGGTG-3' (SEQ ID NO: 8) and primer 3: 5'-AGACACGCACCACCCGGGATCCGCCGGGTCGGCCAGG-3' (SEQ ID NO: 9)/primer 4: 5'-GTGGGAGGTTTTTTCTCTAGACTAGTCT-3' (SEQ ID NO: 10) were used for the reactions. The two PCR fragments obtained were connected by amplification using primer 1 and primer 4. This PCR fragment was confirmed by sequencing, digested with EcoRI and NotI, and inserted into pMXI and pME18S.

The delN mutant has a deletion in the N finger (lacking amino acids 263 to 287). Two PCR fragments comprising this deletion were generated using GATA3 cDNA as a template. The primer combinations of primer 1/primer 5: 5'-GGTAGAGTCCCTCCCTGCCTTCTGTGCTG-3' (SEQ ID NO: 11) and primer 6: 5'-GCAGGGAGGGACTCTACCATAAAATGAATGGGCAG-3' (SEQ ID NO: 12)/primer 4 were used for the reactions. The two PCR fragments were connected by amplification using primer 1 and primer 4. This PCR fragment was confirmed by sequencing, digested with EcoRI and NotI, and inserted into pMXI and pME18S.

The delC mutant has a deletion in the C finger (lacking amino acids 304 to 325). Two PCR fragments comprising this deletion were generated using GATA3 cDNA as a template. The primer combinations of primer 1/primer 7: 5'-TTCATAGTCAGGGGTCGACAGCCTTCGCTTGGGCT-3' (SEQ ID NO: 13) and primer 8: 5'-AAGCGAAGGCTGTCGACCCCTGACTATGAAGAAAGAAGG-3' (SEQ ID NO: 14)/primer 4 were used for the reactions. The two PCR fragments were connected by amplification using primer 1 and primer 4. This PCR fragment was confirmed by sequencing, digested with EcoRI and NotI, and inserted into pMXI and pME18S.

The vectors generated as described above which contained genes encoding GATA3 and GATA3 mutant proteins were expressed in COS cells. The cell extracts were subjected to SDS-PAGE electrophoresis. Each of wild type GATA3 and GATA3 mutant proteins was detected at the expected molecular weight size (data not shown).

### [Example 2] The specificity of the GATA3 protein for the HSS2 sequence and GATA3 binding sequences within the transcription regulatory regions of genes

Cell extracts from COS cells expressing GATA3 were subjected to electrophoretic mobility shift assay (EMSA) using a probe comprising the HSS2 sequence (HSSGATA sequence). The results are shown in Fig. 5. The protein complex which binds to the probe comprising the HSS2 sequence disappeared by excessive addition of unlabeled DNA having the same sequence or a GATA3 binding sequence within the gene transcription regulatory region (TCRαGATA sequence: GATA sequence of the enhancer of T cell receptor α chain) due to competitive inhibition. A mutated HSS2 sequence did not induce inhibition, revealing that the specific sequence was recognized. Anti-GATA3 antibody specific super shift was observed, revealing that the protein in the complex was GATA3.

The GATA3 complex was also detected by EMSA using a probe comprising the GATA3 binding sequence within the gene transcription regulatory region. The complex competitively disappeared by the HSS2 sequence comprising the GATA sequence and the GATA3 binding sequence within the gene transcription regulatory region same as the probe. A mutated HSS2 sequence did not cause inhibition, revealing that the GATA3 complex was recognizing the specific sequence.

### [Example 3] Identification of the region in the GATA3 protein required for binding to the GATA3 binding sequence

Gel shift assays were conducted using the GATA3 mutant proteins and wild type GATA3 protein expressed in COS cells obtained in Example 1 and double stranded oligonucleotides comprising the HSS2 sequence (HSS2 on top panel of Fig. 1, the sense strand is shown in SEQ ID NO: 3), the IL-5 promoter sequence comprising a GATA3 binding sequence (IL-5 promoter on top panel of Fig. 1, the sense strand is shown in SEQ ID NO: 4), and the TCRα enhancer sequence comprising a GATA3 binding sequence (TCRα enhancer on top panel of Fig. 1, the sense strand is shown in SEQ ID NO: 5).

The gel shift assays were conducted by radio-labeling the above synthetic oligonucleotides with ³²P according to conventional methods. The results are shown in Fig. 1. Wild type (WT) GATA3 protein and delTA mutant protein bound to the above-mentioned three types of GATA3 binding sequences; however, the delN mutant bound to only the HSS2 sequence. Moreover, the delC mutant did not bind to any of the three types of GATA3 binding sequences. These results indicated that binding of the GATA3 protein to GATA3 binding sequences other than the HSS2 sequence required both the C and N finger regions of the GATA3 protein, which is consistent with previously reported findings. On the other hand, the results further indicated that binding of the GATA3 protein to the HSS2 sequence requires the C finger region of the GATA3 protein, which, in turn, suggests that its binding behavior is different from that for other GATA3 binding sequences.

Further research conducted by the present inventors revealed that binding of the GATA3 protein to the HSS2 sequence has lower affinity as compared to binding of GATA3 to other GATA3 binding sequences according to the results obtained by affinity measurement experiments using gel shift assays (Fig. 6). There are no other reports except this finding by the present inventors which describe a GATA3 binding sequence with different binding affinity from other GATA3 binding sequences. Accordingly, this finding suggests that only the HSS2 sequence, which is involved in controlling chromatin remodeling, is a low affinity binding sequence among the GATA3 binding sequences.

Therefore, a GATA3 binding sequence with high affinity (*e.g.*, the sequence within the IL-5 promoter sequence, SEQ ID NO: 4; and the sequence within the TCRα enhancer sequence, SEQ ID NO: 5) can be used effectively at low concentrations as a GATA3 decoy that specifically inhibits the low affinity binding of the GATA3 protein to the HSS2 sequence, which is involved in chromatin remodeling, and that does not inhibit the high affinity binding of the GATA3 protein to GATA3 binding sequences other than the HSS2 sequence.

### [Example 4] The effect of the GATA3 protein and its mutants prepared above on chromatin remodeling

The effect of the GATA3 protein and its mutants on chromatin remodeling was examined using a DNase I hypersensitivity assay. A DNase I hypersensitivity assay utilizes the fact that specific fragments can be detected after chromatin remodeling has occurred to allow DNase I to approach its site of action in the genome.

Herein, the DNase I hypersensitivity assay was conducted according to the method described by Takemoto, N., *et al.* (1998, supra). A Phoenix Eco packaging cell line was provided by Dr. Kitamura (The Institute of Medical Science, The University of Tokyo). The plasmids were introduced into the Phoenix Eco packaging cell line using lipofectamine plus (Gibco BRL).

Naive T cells from DO11.10 TCRαβ-transgenic mice (Murphy, K. M., *et al.,* Science 250: 1720 (1990)) were collected by a cell sorter (see Takemoto, N., *et al.,* Int. Immunol. 10: 1981 (1998)) using CD4 expression as an index. The obtained naive T cells were stimulated with peptide antigen derived from chicken ovalbumin and with antigen presenting cells (irradiated mouse splenocytes). One day later, activated T cells were cultured in retrovirus-containing medium supplemented with polyburene (0.5 µg/ml) in order to infect with retrovirus. At the same time, the culture plate was centrifuged at 1800 rpm at 32°C for 1 hour. The procedure was repeated once the next day. The culture was continued in the presence of anti-IL-4 antibody and IL-12, which induces differentiation into Th1. GFP-expressing cells were separated by a cell sorter 7 days after antigen stimulation. The culture was continued for another 2 weeks in the presence of IL-12 and anti-IL-4 antibody while stimulating with antigen every one week.

The obtained infected naive T cells were suspended in 0.5% NP-40-containing RSB buffer (10 mM Tris, pH 7.4, 10 mM NaCl, and 5 mM MgCl₂), and ruptured using a homogenizer to obtain cell lysate. Nuclei were extracted from the lysate. The obtained nuclei (1 x 10⁷ each) were suspended in 100 µl RSB buffer, and DNase I (Worthington Biochemical, Freehold, NJ) was added thereto at concentrations of 0, 5, 10, 15, and 20 µg/ml and incubated at 37°C for 12 min for DNase I reaction. The reaction was stopped by adding 100 µl of 2x reaction stopping buffer (1% SDS, 20 mM Tris, pH 7.4, 600 mM NaCl, 10 mM EDTA, and 50 µg/ml proteinase K) and the reaction mixture was vigorously shaken at 37°C overnight to digest proteins in the mixture. DNA was purified from the mixture by ammonium acetate precipitation, phenol-chloroform extraction, and ethanol precipitation. The DNA was digested with ScaI, electrophoresed on 0.8% agarose gel, and then blotted onto a membrane. The DNA was then hybridized with α³²P ATP-labeled probe (genomic DNA fragment digested with BamHI and NspV corresponding to about 7.0 kb and about 6.7 kb of IL-4 gene transcription initiation site) under conditions generally used for the Southern blot method. The results are shown in Fig. 2. HSS3 digested fragments were detected in all genomic DNA obtained from uninfected cells, wild type GATA3 protein-expressing cells, and all GATA3 mutant protein-expressing cells. However, Th2-specific DNase I hypersensitive sequences HSS2 and HSS1 digested fragments which were specific to Th2 cells undergoing chromatin remodeling were not detected in the genomic DNA of uninfected cells but detected in wild type GATA3 protein-expressing cells. Moreover, concerning infected cells expressing GATA3 mutant proteins, HSS2 and HSS1 digested fragments were detected in cells expressing the delN mutant in a fashion similar to the cells expressing wild type GATA3 protein; however, these fragments were not detected in cells expressing the delTA and delC GATA3 mutant proteins. These results agreed with the results of the gel shift assays in Example 2 which showed that the region within the GATA3 protein required for binding to the HSS2 sequence responsible for chromatin remodeling was the C finger region. Although it was indicated that the delTA mutant could bind to the HSS2 sequence, it lost the region required for chromatin remodeling because HSS2 and HSS1 digested fragments were not detected in delTA mutant-expressing cells. Namely, it was revealed that the delTA mutant can function as a dominant negative mutant in chromatin remodeling induced by the binding of GATA3 protein to the HSS2 sequence.

Moreover, similar experiments were conducted using ³²P-labeled probe for IL-4 coding region to detect the DNase I hypersensitive region within each IL-4 coding region. Again, results obtained for the delN GATA3 mutant protein were similar to those from wild type GATA3 protein while the delTA and delC GATA3 mutant proteins did not reveal the same result as the wild type GATA3 protein did (Fig. 3). These results also indicated that the delTA GATA3 mutant protein was able to bind to the HSS2 sequence, though it lost the region required for chromatin remodeling. Accordingly, these results support the above-mentioned speculation that the delTA mutant can function_as a dominant negative mutant in chromatin remodeling induced by the binding of the GATA3 protein to the HSS2 sequence and the delC GATA3 mutant protein cannot induce chromatin remodeling due to its inability to bind to the HSS2 sequence.

### [Example 5] The effect of GATA3 protein and its mutants prepared above on Th2 cell differentiation

Naive T cells obtained in a similar manner to that in Example 3 were differentiated into Th1 cells by IL-11 stimulation (see Takemoto N, *et al.,* Int. Immunol. 10: 1981 (1998)). The differentiated Th1 cells were infected with each of the above-mentioned viruses which encode a GATA3 mutant or wild type GATA3 protein in a similar manner to that in Example 3. IFNγ, IL-4, and IL-5 expression levels in the infected cells were measured by flow cytometric analysis. The infected cells were collected and activated by PMA (50 ng/ml) and ionomycin (500 ng/ml) for 6 hours. Monensin (2 µM) was added 2 hours before the recovery of cells and cells were cultured. Cells were fixed in 4% paraformaldehyde at 37°C for 5 minutes and washed. Cells were then suspended in 0.5% Triton X-100-containing lysis buffer (50 mM Tris, 150 mM NaCl, 5 mM EDTA, and 0.02% NaN₃, pH7.5) and incubated on ice for 10 minutes to permeabilize the cell membrane. Blocking was performed using PBS containing 3% BSA and 0.1% NaN₃ to inhibit non-specific adsorption of antibody (30 minutes incubation on ice). Cells were stained with FITC-labeled anti-IFNγ antibody (XMG1.2), phycoerythrin-labeled anti-IL-4 antibody (11B11, R&D Systems, Minneapolis, MN), or PE-labeled anti-IL-5 antibody (TRFK5; Pharmingen) for 60 minutes. Flow cytometric analysis was conducted after unbound antibodies were removed by washing. The analysis results are shown in Fig. 4. The far right column of Fig. 4 shows the results obtained from Th2 cells differentiated by IL-4 stimulation which were treated and analyzed as described above. It is revealed that IFNγ is not expressed but IL-4 and IL-5 are expressed at higher levels in Th2 cells as compared to the results obtained from Th1 cells differentiated from uninfected cells (left column). On the other hand, it is revealed that Th1 cells have high IFNγ expression and very low IL-4 and IL-5 expression. Moreover, Th1 cells expressing the wild type GATA3 protein have lower IFNγ expression as compared to Th1 cells and, furthermore, expression of IL-4 and IL-5 is induced. Accordingly, it is found that the expression of wild type GATA3 protein in Th1 cells induces Th2 cytokine expression and results in changes in the phenotype of Th1 cells to that of Th2 cells.

Among the 34 kinds of GATA3 mutant proteins used for this experiment, the delN GATA3 mutant protein had the same function as the wild type GATA3 protein with respect to IFNγ and IL-4 and enabled Th2 cell phenotypic representation. Unlike wild type GATA3 protein, this mutant protein cannot induce IL-5, which may be because the binding of the GATA3 protein to the GATA3 binding sequence within the promoter sequence is required for sufficient IL-5 expression. Recall, the delN mutant protein has lost the ability to bind to the GATA3 binding sequence within the promoter sequence as described above. On the other hand, such phenotypic changes, *i.e.*, changes in IFNγ, IL-4, and IL-5 expression patterns, were not observed by the expression of the delC and delTA GATA3 mutant proteins. The results obtained from the gel shift assays and DNase I hypersensitivity assays revealed that the delC GATA3 mutant protein cannot induce chromatin remodeling due to the inability to bind to the HSS2 sequence and the delTA GATA3 mutant protein cannot induce chromatin remodeling, although it can bind to the HSS2 sequence. Thus, the above flow cytometric analyses reconfirmed that the chromatin remodeling is essential for differentiation into Th2 cells.

Furthermore, a GATA3 mutant protein having a partial deletion in the N finger region (lacking amino acid residues 280 to 288) was also examined by all the experiments described above and showed the same results as the results obtained from the delN mutant protein, although the data is not shown. Moreover, the mutant (V264G) having a substitution of one amino acid residue within the N finger region (the corresponding residue in GATA-1 protein is essential to mediate contact with FOG-1 (Crispino, J. D. *et al.,* Mol. Cell. 3: 219 (1999)) showed the same results as the results obtained by the wild type GATA3 protein. On the other hand, the mutant in which 3 amino acid residues from 304 to 306 located between the N and C finger regions were substituted with AAA lowered IL-5 production but showed about the same results as the results obtained from wild type GATA3 protein regarding IFNγ and IL-4. Moreover, the mutant lacking amino acids 309 to 345, which contained the C finger region, also showed the same results as the results obtained from the delC GATA3 mutant protein.

Summarizing the above results, the delC GATA3 mutant protein lacks the ability to bind to the HSS2 sequence which is required for chromatin remodeling and also lacks the ability to bind to other GATA3 binding sequences besides HSS2. Accordingly, the C finger region of GATA3 protein is essential for binding not only to the HSS2 sequence but also to all GATA3 binding sequences. Since the delN GATA3 mutant protein is able to bind to the HSS2 sequence and induce phenotypic changes characteristic of Th2 cells, the N finger region of the GATA3 protein is not involved in binding to the HSS2 sequence or chromatin remodeling, but it is essential for binding to GATA3 binding sequences other than HSS2. Accordingly, among GATA3 binding sequences, the HSS2 sequence alone requires only the C finger region of the GATA3 protein for binding to the GATA3 protein, and other GATA3 binding sequences require both the C and N finger regions. Moreover, the delTA GATA3 mutant protein bound to all GATA3 binding sequences tested including HSS2, however, it does not induce chromatin remodeling nor phenotypic changes of Th2 cells. Accordingly, the delTA GATA3 mutant protein is a dominant negative mutant of the GATA3 protein for chromatin remodeling. The delTA GATA3 mutant protein has lost part of the transactivation region and this lost region contains the region necessary for chromatin remodeling after binding to the HSS2 sequence. This discussion easily leads those skilled in the art to consider the following: since a GATA3 mutant protein having deletions in both the transactivation and N finger regions binds to the HSS2 sequence but does not induce chromatin remodeling and it cannot bind to GATA3 binding sequences other than HSS2, this mutant can be a GATA3 mutant protein which has no effect on intracellular mechanisms (*e.g.*, transcription activation of IL-5, TCRα, and such) induced by GATA3 protein other than chromatin remodeling and is a dominant negative mutant which inhibits only Th2 cell differentiation specifically and effectively.

While both the C and N finger regions are necessary for binding to other GATA3 binding sequences, only the C finger region within the GATA3 protein is necessary for binding of the GATA3 protein to the HSS2 sequence, and this binding has relatively low affinity. Therefore, it is further suggested that an oligonucleotide comprising a GATA3 binding sequence having higher binding affinity is very useful as a decoy that specifically inhibits the lower affinity binding of the GATA3 protein to the HSS2 sequence, which is involved in chromatin remodeling.

### [Example 6] Comparison of the binding affinity of the GATA3 protein to the HSS2 sequence (CNS-1) and the GATA3 binding sequence within the transcription regulatory region of genes (TCRα enhancer sequence) using gel shift assays

A competitive binding inhibition experiment was performed by gel shift assay using wild type GATA3 protein expressed in COS cells obtained in Example 1, a double stranded oligonucleotide comprising the HSS2 sequence ("HSS2", top right panel of Fig. 6, sense strand is shown in SEQ ID NO: 3), and a double stranded oligonucleotide comprising the TCRα enhancer sequence containing a GATA3 binding sequence ("TCRα enhancer", top left panel of Fig. 6, sense strand is shown in SEQ ID NO: 5). The gel shift assay was performed according to conventional methods using the above synthetic oligonucleotide probes labeled with ³²P (Fig. 6). Wild type GATA3 protein formed a complex with the ³²P-labeled TCRα enhancer sequence containing the GATA3 binding sequence and the complex was detected by electrophoresis (top left panel of Fig. 6, lane 1). Unlabeled DNA comprising the TCRα enhancer sequence which had the GATA3 binding sequence of the probe was added to the reaction mixture at 50 and 100 times that of the probe. Unlabeled DNA comprising the HSS2 sequence was added at 50, 100, 200, 400, and 800 times (bottom left panel of Fig. 6). In contrast, by gel shift assay using ³²P-labeled HSS2 sequence probe, competitive inhibition of binding was conducted by adding 5, 10, 20, 50, and 100 times of unlabeled DNA comprising the TCRα enhancer sequence (top right panel of Fig. 6) and 5, 10, 20, 50, and 100 times of unlabeled DNA comprising the HSS2 sequence (bottom right panel of Fig. 6).

As a result, the same sequence, other than the HSS2 sequence, showed competitive inhibition at 50 times the amount but the HSS2 sequence did not inhibit completely even at 200 times the amount, by gel shift assay using as a probe the GATA3 binding sequence within the transcription regulatory region of the gene. In contrast, using the HSS2 sequence as a probe, the GATA3 binding sequence within the transcription regulatory region of the gene at 50 times the amount inhibits almost all, but the HSS2 sequence does not inhibit completely even at 100 times the amount. These results revealed that the binding affinity of the GATA3 protein to the GATA3 binding sequence within the transcription regulatory region of the gene is higher than that to the HSS2 sequence.

### Industrial Applicability

The present invention provides GATA3 decoys and GATA3 mutant proteins for treating and/or preventing Th2 cytokine-related allergic diseases. The decoys and GATA3 mutant proteins of the present invention selectively suppress chromatin remodeling in the Th2 cytokine gene cluster and effectively suppress Th2 cytokine expression without affecting intracellular functions of the GATA3 protein other than that for Th2 cytokines. Therefore, pharmaceutical compositions comprising such decoys or mutant proteins as active ingredients for treating and/or preventing Th2 cytokine-related allergic diseases, and methods for treating and/or preventing Th2 cytokine-related allergic diseases comprising administering such a composition are extremely effective.

## Claims

1. A GATA3 decoy for treating and/or preventing Th2 cytokine-related allergy diseases.

2. The GATA3 decoy according to claim 1, wherein the decoy suppresses chromatin remodeling within a Th2 cytokine gene cluster region by inhibiting the binding of a GATA3 protein to an HSS2 sequence in the genome and thereby suppresses the production of one or more Th2 cytokines in T cells.

3. The GATA3 decoy according to claim 2, wherein the decoy does not inhibit the binding of a GATA3 protein to a GATA3 binding sequence in a transcription regulatory region of a gene which is transcriptionally activated by the GATA3 protein.

4. The GATA3 decoy according to claim 2, wherein the decoy inhibits at a lower level the binding of a GATA3 protein to a GATA3 binding sequence in a transcription regulatory region of a gene which is transcriptionally activated by the GATA3 protein as compared to the binding of the GATA3 protein to an HSS2 sequence in the genome.

5. The GATA3 decoy according to claim 1, wherein the decoy specifically inhibits the binding of a GATA3 protein to an HSS2 sequence in the genome.

6. The GATA3 decoy according to any one of claims 1 to 5, wherein the decoy comprises a double stranded oligonucleotide, or derivative thereof, comprising a sequence set forth in SEQ ID NO: 3, 4, 5, or 6.

7. The GATA3 decoy according to any one of claims 1 to 6, wherein the decoy effectively inhibits the binding of a GATA3 protein to an HSS2 sequence in a T cell genome, further wherein the decoy can be administered at a concentration such that it inhibits at a lower level the binding of the GATA3 protein to a GATA3 binding sequence in a transcription regulatory region of a gene which is transcriptionally activated by the GATA3 protein as compared to the binding of the GATA3 protein to the HSS2 sequence in the genome.

8. A GATA3 mutant protein or derivative thereof, wherein:
a) the amino acid sequence of the C finger region within a wild type GATA3 protein is conserved; or
b) one or more amino acids are deleted, substituted, or inserted in the amino acid sequence of the C finger region within the wild type GATA3 protein yet the binding ability to HSS2 sequence is maintained.

9. The GATA3 mutant protein or derivative thereof according to claim 8, wherein one or more amino acids are deleted, substituted, or inserted in the amino acid sequence of the transactivation region within the wild type GATA3 protein such that the ability of the GATA3 protein to induce chromatin remodeling is lost.

10. The GATA3 mutant protein or derivative thereof according to claim 9, wherein at least ten amino acid residues are deleted in the amino acid sequence of the transactivation region within the wild type GATA3 protein.

11. The GATA3 mutant protein or derivative thereof according to any one of claims 8 to 10, wherein one or more amino acids are further deleted, substituted, or inserted in the amino acid sequence of the N finger region within the wild type GATA3 protein such that the ability to bind to a DNA comprising a GATA3 binding sequence within a transcription regulatory region of a gene which is transcriptionally activated by the GATA3 protein is lost.

12. The GATA3 mutant protein or derivative thereof according to any one of claims 8 to 10, wherein at least one amino acid residue is deleted in the amino acid sequence of the N finger region within the wild type GATA3 protein.

13. A GATA3 mutant protein or derivative thereof, wherein at least ten amino acid residues are deleted in the amino acid sequence of the transactivation region and at least one amino acid residue is deleted in the amino acid sequence of the N finger region within the wild type GATA3 protein.

14. A mouse GATA3 mutant protein or derivative thereof, wherein amino acids 29 to 168 in the amino acid sequence of the mouse wild type GATA3 protein set forth in SEQ ID NO: 1 are deleted.

15. A human GATA3 mutant protein or derivative thereof, wherein amino acids 29 to 169 in the amino acid sequence of the human wild type GATA3 protein set forth in SEQ ID NO: 2 are deleted.

16. The GATA3 mutant protein or derivative thereof according to claim 14, wherein at least amino acids 280 to 287 in the amino acid sequence of the N finger region within the mouse wild type GATA3 protein set forth in SEQ ID NO: 1 are further deleted.

17. The human GATA3 mutant protein or derivative thereof according to claim 15, wherein at least amino acids 281 to 288 in the amino acid sequence of the N finger region within the human wild type GATA3 protein set forth in SEQ ID NO: 2 are further deleted.

18. The GATA3 mutant protein or derivative thereof according to any one of claims 8 to 17, wherein the protein or derivative has the ability to bind to the HSS2 sequence but suppress Th2 cytokine production in T cells.

19. The GATA3 mutant protein or derivative thereof according to claim 18, wherein the ability to bind to a DNA comprising a GATA3 binding sequence within a transcription regulatory region of a gene which is transcriptionally activated by a GATA3 protein is lost.

20. A nucleic acid encoding the GATA3 mutant protein according to any one of claims 8 to 19.

21. A vector comprising the nucleic acid according to claim 20.

22. The vector according to claim 21, wherein the vector is an expression vector.

23. A host cell comprising the vector according to claim 21 or 22.

24. A pharmaceutical composition for treating and/or preventing Th2 cytokine-related allergic diseases, wherein the composition suppresses the production of one or more Th2 cytokines upon administered to a mammal by suppressing chromatin remodeling within a Th2 cytokine gene cluster region due to the inhibition of binding of a GATA3 protein to the HSS2 sequence in the genome.

25. The pharmaceutical composition according to claim 24, wherein the pharmaceutical composition comprises one or more of the GATA3 decoys according to any one of claims 1 to 7.

26. The pharmaceutical composition according to claim 24, wherein the pharmaceutical composition comprises one or more of the GATA3 mutant proteins according to any one of claims 8 to 19.

27. The pharmaceutical composition according to claim 24, wherein the pharmaceutical composition comprises one or more of the nucleic acids encoding a GATA3 mutant protein according to claim 20.

28. The pharmaceutical composition according to claim 24, wherein the pharmaceutical composition comprises one or more of the vectors according to claim 21 or 22.

29. A method of screening for a drug for treating and/or preventing Th2 cytokine-related allergic diseases, wherein the method comprises the steps of:
a) incubating a wild type GATA3 protein or a GATA3 mutant protein which possesses the ability to bind to an HSS2 sequence with a dsDNA or derivative thereof which comprises the HSS2 sequence, with or without a test compound, under conditions that allow for the binding of the wild type GATA3 protein or the GATA3 mutant protein and the dsDNA or derivative thereof;
b) detecting the binding of the wild type GATA3 protein or the GATA3 mutant protein to the dsDNA or derivative thereof; and
c) identifying a test compound which is able to inhibit said binding.

30. The method according to claim 29, wherein the method further comprises the steps of:
d) incubating the wild type GATA3 protein or a GATA3 mutant protein which possesses the ability to bind to a GATA3 binding sequence within a transcription regulatory region of a gene with a dsDNA or derivative thereof which comprises the GATA3 binding sequence within the transcription regulatory region of the gene, with or without the test compound, under conditions that allow for the binding of the wild type GATA3 protein or GATA3 mutant protein and the dsDNA or derivative thereof ;
e) detecting the binding of the wild type GATA3 protein or GATA3 mutant protein to the dsDNA or derivative; and
f) identifying the test compound which inhibits the binding detected in step e) at a lower level as compared to the binding detected in step b).
